# EUROPÄISCHE PATENTSCHRIFT

(11) **EP 2 331 181 B1**
(45) Veröffentlichungstag und Bekanntmachung des Hinweises auf die Patenterteilung: **20.11.2013**
(21) Anmeldenummer: 09777969.8
(22) Anmeldetag: 19.08.2009
(51) Int. Cl.: A61M 25/00

(54) **KATHETEREINRICHTUNG**
CATHETER DEVICE
DISPOSITIF CATHETER

(30) Priorität: 20.08.2008 DE 102008038477; 13.11.2008 DE 102008057255
(43) Veröffentlichungstag der Anmeldung: 15.06.2011
(73) Patentinhaber: Krombach, Gabriele, 6294 BJ Vijlen (NL); Friebe, Michael, 45657 Recklinghausen (DE)
(72) Erfinder: Krombach, Gabriele, 6294 BJ Vijlen (NL); Friebe, Michael, 45657 Recklinghausen (DE)
(74) Vertreter: Von Rohr
(86) Internationale Anmeldenummer: PCT/EP2009/006004
(87) Internationale Veröffentlichungsnummer: WO 2010/020404

(56) Entgegenhaltungen:
- WO-A1-2007/131516
- WO-A2-99/49926
- WO-A2-03/009884

## Beschreibung

Die Erfindung betrifft eine Kathetereinrichtung für perkutane Eingriffe, insbesondere für Injektionen, Biopsien oder dergleichen, mit einem Außenkatheter und einem Werkzeug für den Eingriff, insbesondere einer Injektionsnadel, einer Biopsiezange, Elektroden oder dergleichen, am proximalen Ende der Kathetereinrichtung, wobei das Werkzeug in einem Zuführzustand im Außenkatheter aufgenommen und die Werkzeugspitze des Werkzeugs in dem Zuführzustand im Außenkatheter versenkt oder mit dem proximalen Ende des Außenkatheters ausgefluchtet ist, wobei das Werkzeug relativ zum Außenkatheter derart bewegbar ist, daß zumindest die Werkzeugspitze in einem Eingriffszustand über das proximale Ende des Außenkatheters übersteht und wobei ein Begrenzungselement zur Eingriffstiefenbegrenzung des Werkzeugs beim Eingriff vorgesehen ist.

Die Fortschritte in der Molekularbiologie und Gentechnologie der letzten Jahre haben zur Entwicklung verschiedenartiger therapeutischer Substanzen geführt, die zum Beispiel die Angioneogenese im ischämisch geschädigten Myokard oder die Ansiedlung von omnipotenten Stammzellen und Differenzierung zu Myozyten in Myokardnarben anstreben. Klinische Untersuchungen bei Patienten zeigten, daß therapeutische Gewebekonzentrationen derartiger Substanzen nur nach direkter Injektion in das Gewebe erzielt werden konnten. Dabei läßt sich eine Injektion derartiger Substanzen ohne weiteres bei einer Operation am offenen Herzen durchführen. Derartige Operationen sind jedoch stets mit einem nicht unerheblichen Aufwand und einem entsprechenden Risiko verbunden. Einfacher erscheint in diesem Zusammenhang die perkutane Injektion mittels einer Kathetereinrichtung. Problematisch ist hierbei jedoch, daß bei einer perkutanen Injektion am Katheter eine entsprechende Nadel vorgesehen werden muß und beim Einbringen des Katheters in den Körper beispielsweise an einer Leiste oder einer Armbeuge bis in das Herz das Vorführen durch die Arterien nicht zu einer Verletzung der Gefäße führen darf. Ein weiteres Problem bei der perkutanen Injektion ergibt sich dadurch, daß es unbeabsichtigt passieren kann, daß bei der Injektion zu tief in das Herzgewebe eingestochen und/oder das Herzgewebe sogar durchstochen wird. Das ungewollte Durchstechen des Herzgewebes birgt die Gefahr des Vollaufens des Herzbeutels mit Blut und folgender Kompression des Herzens mit möglicher Todesfolge in sich.

Ähnliche Probleme ergeben sich beispielsweise bei perkutanen Gewebeentnahmen, bei denen eine Biopsiezange mittels eines Katheters an die Entnahmestelle gebracht wird.

Aus der WO 2007/131516 A1 ist eine Kathetereinrichtung für perkutane Eingriffe der vorgenannten Art bekannt. Bei der bekannten Kathetereinrichtung ist ein Begrenzungselement zur Einstichtiefenbegrenzung einer Nadel der Kathetereinrichtung vorgesehen, das sicherstellt, daß die Nadel nur bis zu einer genau vorgegebene Länge in das betreffende Gewebe eindringen kann. Das Begrenzungselement steht im Eingriffszustand in radialer Richtung über den Außenkatheter über, wobei der Überstand ein Mehrfaches des Außendurchmessers des Außenkatheters betragen kann. Durch ein derart großes Begrenzungselement soll letztlich sichergestellt werden, daß weder die Nadel noch der Außenkatheter oder ein innerhalb des Außenkatheters bewegbar geführter Innenkatheter, der mit der Nadel verbunden ist, das Gewebe durchstechen können. Das Begrenzungselement weist federnde Begrenzungsarme auf, die im Eingriffszustand gegen das Gewebe anliegen und eine Begrenzung der Einstichtiefe bewirken. Im Zuführzustand sind die Begrenzungsarme an den Innenkatheter angelegt und entgegen der Federkraft in den Außenkatheter aufgenommen. Beim Ausfahren des Werkzeugs werden dann die Begrenzungsarme ausgefahren, wobei sich die Begrenzungsarme bananenschalenförmig in radialer Richtung nach außen hin öffnen.

Aufgabe der vorliegenden Erfindung ist es, eine Kathetereinrichtung der eingangs genannten Art zur Verfügung zu stellen, die sich in einfacher Weise in den Körper einführen läßt und eine wirkungsvolle Eingriffstiefenbegrenzung des Werkzeugs sicherstellt, ohne daß es zu einer Gewebebeschädigung durch das Begrenzungselement kommen kann.

Zur Lösung der vorgenannten Aufgabe ist bei einer Kathetereinrichtung der eingangs genannten Art gemäß einer ersten Ausführungsform der Erfindung vorgesehen, daß das Begrenzungselement im Zuführzustand eine nicht-expandierte Grundform aufweist und daß das Begrenzungselement durch Stauchen von der Grundform in eine expandierte Begrenzungsform im Eingriffszustand überführbar ist, wobei beim Stauchen eine relative Längenänderung des Begrenzungselementes bewirkt wird und wobei das Begrenzungselement beim Stauchen in radialer Richtung nach außen hin und/oder in proximaler Richtung expandiert und wenigstens eine in radialer und/oder proximaler Richtung über den Außenkatheter überstehende Begrenzungsfläche zur Anlage gegen ein Körpergewebe im Eingriffszustand ausbildet.

Das Begrenzungselement ist ein separates Bauteil der erfindungsgemäßen Kathetereinrichtung. Der Erfindung liegt an dieser Stelle der Grundgedanke zu Grunde, ein Begrenzungselement zur Eingriffstiefenbegrenzung des Werkzeugs, vorzugsweise zur Einstichtiefenbegrenzung einer Nadel, zur Verfügung zu stellen, wobei das Begrenzungselement durch Aufbringen von Druckkräften gestaucht wird, was zur Folge hat, daß sich das Begrenzungselement in radialer Richtung nach außen hin verformt und die Begrenzungsfläche(n) ausbildet, die schließlich beim Zuführen der Kathetereinrichtung in den Körper gegen ein Körpergewebe zur Anlage kommt bzw. kommen. Das Inkontakttreten der Begrenzungsfläche(n) gegen das Gewebe ist von dem behandelnden Arzt als plötzlich auftretender Widerstand beim Zuführen der Kathetereinrichtung in den Körper deutlich spürbar. Die erfindungsgemäße Ausgestaltung des Begrenzungselementes als stauchbarer Körper ermöglicht eine sehr geringe Baugröße der Kathetereinrichtung im Zuführzustand und stellt darüber hinaus sicher, daß das Begrenzungselement im expandierten Eingriffszustand eine Größe aufweist, die eine sichere und störunempfindliche Eingriffstiefenbegrenzung des Werkzeugs gewährleistet. Zudem läßt sich das Begrenzungselement durch Entlastung leicht in die Grundform überführen und der Katheter einfach aus dem Körper wieder entfernen. Im übrigen weist die erfindungsgemäße Kathetereinrichtung einen einfachen konstruktiven Aufbau auf und läßt sich einfacher Weise und kostengünstig herstellen.

Zur Lösung der eingangs genannten Aufgabe ist bei einer weiteren Ausführungsform der Erfindung vorgesehen, daß ein distales Ende des Begrenzungselementes fest mit dem Werkzeug verbunden ist und/oder zusammen mit dem Werkzeug in Längsrichtung des Werkzeuges verschiebbar gelagert ist, daß ein proximales Ende des Begrenzungselementes und das Werkzeug relativ zueinander verschiebbar sind und daß das Begrenzungselement im Bereich zwischen dem distalen Ende und dem proximalen Ende elastisch ausgebildet ist, wobei das Begrenzungselement durch Vorschieben des Werkzeugs aus dem Außenkatheter in radialer Richtung nach außen hin und/oder in proximaler Richtung expandierbar ist und wenigstens eine Begrenzungsfläche zur Begrenzung der Eingriffstiefe ausbildet.

Damit es beim Vorschieben des Werkzeugs zu einem Expandieren des Begrenzungselementes kommen kann, ist dieses lediglich an seinem distalen Ende fest mit dem Werkzeug verbunden. Beim Überführen von dem Zuführzustand in den Eingriffszustand kommt es zur Expansion des Begrenzungselementes, wobei sich die Länge des Begrenzungselementes verkürzt und der elastische Bereich des Begrenzungselementes nach außen biegt. Beispielsweise kann vorgesehen sein, daß das Begrenzungselement federelastische Begrenzungsschenkel aufweist, die am distalen Ende des Begrenzungselementes mit dem Werkzeug oder einem das Werkzeug tragenden Innenkatheter und am proximalen Ende miteinander verbunden und verschiebbar zum Werkzeug angeordnet sind. Im Zuführzustand sind die Begrenzungsschenkel in den Außenkatheter eingezogen und damit aufgrund ihrer Federeigenschaft vorgespannt. Beim Vorschieben des Werkzeugs wird das Begrenzungselement aus dem Außenkatheter herausgeschoben, was automatisch zum Entfalten der Begrenzungsschenkel führt. Beim Entfalten der Begrenzungsschenkel zieht sich das Begrenzungselement zusammen und bildet die Begrenzungsform im Eingriffszustand aus. Da die Begrenzungsschenkel am proximalen Ende des Begrenzungselementes miteinander verbunden sind, wird eine hohe Stabilität des Begrenzungselementes bei der Eingriffstiefenbegrenzung sichergestellt.

Es versteht sich, daß Merkmale der beiden zuvor beschriebenen und anhand der Zeichnung nachfolgend näher erläuterten alternativen Ausführungsformen der Erfindung auch bedarfsweise miteinander kombiniert werden können, ohne daß dies im einzelnen beschrieben ist.

Weitere zweckmäßige Ausgestaltungen und Vorteile sind Gegenstand der nachfolgenden Beschreibung von Ausführungsbeispielen der Erfindung unter Bezug auf die Figuren der Zeichnung, wobei die Erfindung nicht auf die in den Figuren dargestellten Ausführungsbeispiele beschränkt ist und wobei
- Fig. 1: eine Längsansicht des vorderen Teils einer erfindungsgemäßen Kathetereinrichtung im Zuführzustand zeigt,
- Fig. 2: eine Längsansicht der in Fig. 1 dargestellten Kathetereinrichtung im Eingriffszustand zeigt,
- Fig. 3: eine perspektivische Ansicht eines Begrenzungselementes der in den Figuren 1 und 2 dargestellten Kathetereinrichtungen in der Grundform zeigt,
- Fig. 4: den vorderen Teil einer weiteren Ausführungsform einer erfindungsgemäßen Kathetereinrichtung in einer perspektivischen Ansicht zeigt,
- Fig. 5: eine Längsansicht des vorderen Teils einer dritten Ausführungsform einer erfindungsgemäßen Kathetereinrichtung im Zuführzustand zeigt,

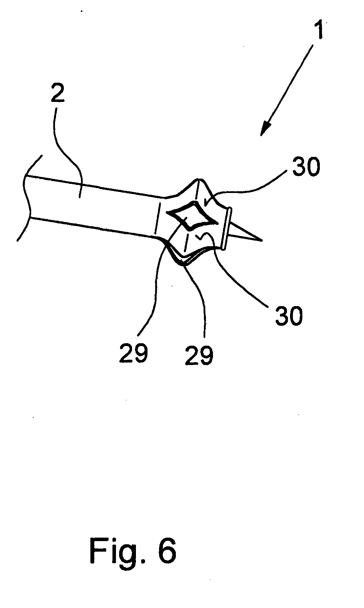

- Fig. 6: die in Fig. 5 dargestellte Kathetereinrichtung beim Stauchen des Außenkatheters zeigt,

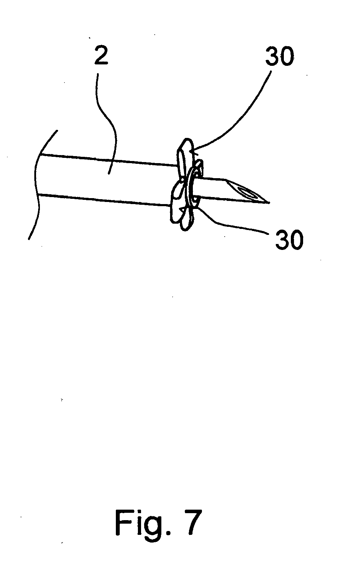

- Fig. 7: die in Fig. 5 dargestellte Kathetereinrichtung im Eingriffszustand zeigt,
- Fig. 8: eine Längsansicht des vorderen Teils einer vierten Ausführungs-form einer erfindungsgemäßen Kathetereinrichtung im Zuführzustand zeigt,
- Fig. 9: eine Längsseitenansicht des vorderen Teils der in Fig. 8 dargestellten Kathetereinrichtung im Eingriffszustand zeigt,
- Fig. 10: eine perspektivische Ansicht der in Fig. 8 dargestellten Kathetereinrichtung schräg von vorne im Eingriffszustand zeigt,
- Fig. 11: eine perspektivische Ansicht des vorderen Teils einer fünften Ausführungsform einer erfindungsgemäßen Kathetereinrichtung im Eingriffszustand zeigt,
- Fig. 12: eine Seitenansicht eines Begrenzungselementes der in Fig. 11 dargestellten Kathetereinrichtung im Zuführzustand zeigt und
- Fig. 13: eine Stirnansicht auf das in Fig. 12 dargestellte Begrenzungselement im Eingriffszustand zeigt.

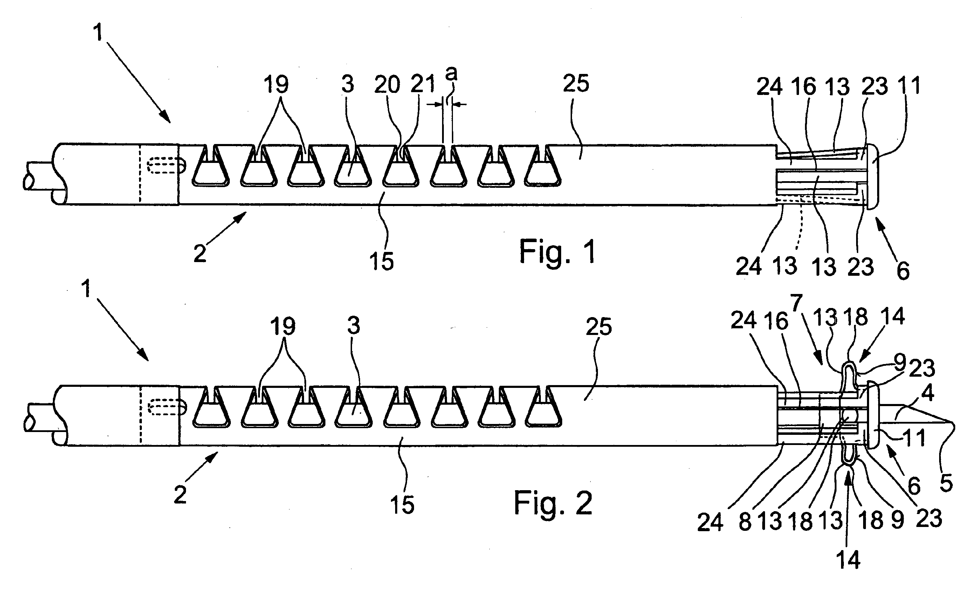

In Fig. 1 ist eine Seitenansicht auf den vorderen Endabschnitt einer Kathetereinrichtung 1 dargestellt. Die Kathetereinrichtung 1 weist einen Außenkatheter 2 und einen im Außenkatheter 2 bewegbar geführten Innenkatheter 3 auf. Am proximalen Ende des Innenkatheters 3 ist eine mit diesem verbundene Nadel 4 als Werkzeug vorgesehen. Die Nadel 4 ist in einem in Fig. 1 dargstellten Zuführzustand der Kathetereinrichtung 1 im Außenkatheter 2 versenkt, wobei das proximale Ende 5 der Nadel 4 mit dem proximalen Ende 6 des Außenkatheters 2 ausgefluchtet ist. Die Nadel 4 ist relativ zum Außenkatheter 2 derart bewegbar geführt, daß in einem Eingriffszustand das proximale Ende 5 der Nadel 4 über das proximale Ende 6 des Außenkatheters 2 übersteht.

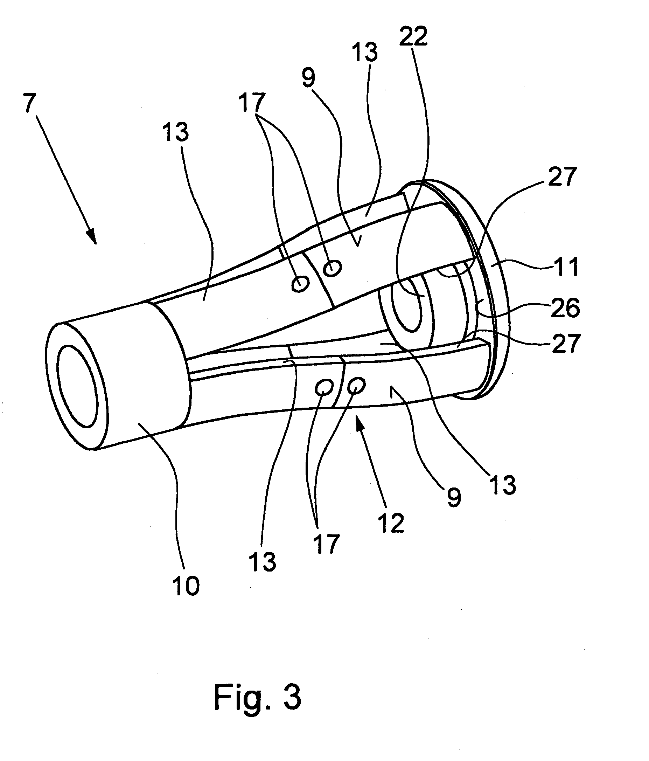

Darüber hinaus weist die Kathetereinrichtung 1 ein Begrenzungselement 7 auf, das in Fig. 1 in einer nicht-expandierten Grundform im Zuführzustand und in Fig. 2 in einer expandierten Begrenzungsform im Eingriffszustand dargestellt ist. Wesentlich dabei ist, daß das Begrenzungselement 7 durch Stauchen von der Grundform in die Begrenzungsform überführbar ist, wobei beim Stauchen eine relative Längenänderung des Begrenzungselements 7 bewirkt wird und wobei das Begrenzungselement 7 beim Stauchen in radialer Richtung nach außen hin expandiert und eine Mehrzahl von in radialer Richtung über den Außenkatheter 2 überstehende Begrenzungsflächen 9 zur Anlage gegen ein Körpergewebe und zur Eingriffstiefenbegrenzung im Eingriffszustand ausbildet. Das Begrenzungselement 7 ist in Fig. 3 perspektivisch im Zuführzustand dargestellt.

Das Begrenzungselement 7 ist im Zuführzustand hülsenförmig und weist einen mit dem Innenkatheter 3 verbundenen distalen Ringabschnitt 10 und einen mit dem Außenkatheter 2 verbundenen proximalen Ringabschnitt 11 auf. Der proximale Ringabschnitt 11 kann in radialer Richtung geringfügig über den Außenkatheter 2 überstehen. Die Ringabschnitte 10, 11 sind durch einen mittleren elastischen Bereich 12 verbunden. Der elastische Bereich 12 wird durch eine Mehrzahl von sich in Längsrichtung bzw. in Richtung der Längsachse des Begrenzungselements 7 erstreckenden Begrenzungsschenkeln 13 gebildet, wobei sich die Schenkel 13 beim Stauchen des Begrenzungselements 7 in radialer Richtung nach außen biegen und die Begrenzungsflächen 9 aufweisende nach außen gewölbte bzw. schlaufenförmige Begrenzungsarme 14 zur Anlage gegen das Körpergewebe im Eingriffszustand ausbilden. Dies ist in Fig. 2 dargestellt. Das Stauchen des Begrenzungselements 7 wird durch Vorschieben des Innenkatheters 3 zusammen mit der Nadel 4 bewirkt, wobei der distale Ringabschnitt 10 zusammen mit dem Innenkatheter 3 in Richtung zu dem am proximalen Ende eines Verlängerungsstücks 15 des Außenkatheters 2 festgesetzten proximalen Ringabschnitt 11 des Begrenzungselements 7 bewegt wird. Der distale Ringabschnitt 10 wird also relativ zu dem proximalen Ringabschnitt 11 in axialer Richtung bewegt, was zum Ausfedern bzw. Herausbiegen der Schenkel 13 durch entsprechende sich in Längsrichtung erstreckende Führungsöffnungen 16 in dem Verlängerungsstück 15 führt. Wird der Innenkatheter 3 nach der Behandlung zusammen mit der Nadel 4 vom Gewebe zurückgezogen und die Kathetereinrichtung 1 damit von dem Eingriffszustand wieder in den Zuführzustand überführt, führt dies zu einer Entlastung des Begrenzungselementes 7 und zu einem Zurückfedern in die in Fig. 3 gezeigte Grundform.

Das Begrenzungselement 7 ist elastisch verformbar, wobei die Rückstellkräfte vorzugsweise ausreichend groß sind, um das Begrenzungselement 7 nach der Druckentlastung bzw. beim Zurückziehen des Innenkatheters 3 automatisch von der Begrenzungsform im Eingriffszustand wieder in die Grundform im Zuführzustand zu überführen.

Bei einer nicht dargstellten alternativen Ausführungsform kann auch ein als luftgefüllter Rollbalg ausgebildetes Begrenzungselement vorgesehen sein, wobei das Begrenzungselement über den Außenkatheter 2 bereichsweise gestülpt ist. Beim Stauchen rollt der Rollbalg dann auf dem Außenkatheter 2 ab und bildet dabei eine wulstartige den Außenkatheter 2 ringförmig umgebende Begrenzungsfläche aus, um eine Einstichtiefenbegrenzung zu gewährleisten.

Im Eingriffszustand können die Begrenzungsarme 14 in radialer Richtung mehr als 3 mm, vorzugsweise ca. 4 mm, über den Außenkatheter 2 überstehen, jeweils mit Bezug auf den Abstand der äußeren Scheitelpunkte 18 der Begrenzungsarme 14 zur äußeren Mantelfläche des Verlängerungsstücks 15. Es versteht sich, daß sich die Begrenzungsarme 14 im Eingriffszustand auch weiter in den den Außenkatheter 2 umgebenen Raum erstrecken können, was letztlich durch die Länge der Schenkel 13 und den möglichen Verstellweg des Innenkatheters 3 festgelegt ist.

Darüber hinaus sind gemäß der in den Figuren 1 und 2 dargestellten Kathetereinrichtung 1 im Eingriffszustand vier voneinander beabstandete Begrenzungsarme 14 vorgesehen. Kommen die Begrenzungsarme 14 im Eingriffszustand zur Anlage gegen das Gewebe, beispielsweise zur Anlage gegen eine Herzinnenwand, wird dies von einem behandelnden Arzt als deutlich spürbarer Widerstand gegen ein weiteres Vorschieben der Nadel 4 in Richtung zum Gewebe wahrgenommen. Es versteht sich, daß auch eine größere oder auch eine kleinere Anzahl von Begrenzungsarmen 14 vorgesehen sein kann. Die Begrenzungsarme 14 erstrecken sich in radialer Richtung, so wie dies in Fig. 2 dargestellt ist. Grundsätzlich können die Begrenzungsarme 14 aber auch nach vorne abgewinkelt oder abgebogen sein und sich ggf. auch über das proximale Ende des Außenkatheters 2 hinaus nach vorne erstrecken. Auch ist es möglich, daß die Begrenzungsarme 14 in Richtung auf das Werkzeug abgebogen oder abgewinkelt sind. Die Begrenzungsarme 14 weisen nach außen gewölbte bzw. abgerundete Begrenzungsflächen 9 aus.

Das Begrenzungselement 7 kann einstückig und aus Kunststoff hergestellt sein, beispielsweise aus Polyurethan, wobei, vorzugsweise, dem Kunststoff Metallpartikel als Zuschlagstoff beigefügt sein können. Die Metallpartikel im Kunststoff führen dazu, daß das Begrenzungselement 7 und insbesondere die Begrenzungsarme 14, im Eingriffszustand mittels MRT-Bildgebung erkennbar sind, was das Zuführen der Kathetereinrichtung 1 zum Eingriffsort erleichtert. Ergänzend und/oder alternativ kann wenigstens ein Begrenzungsarm 14 einen metallischen Marker 17 auf der Außenseite aufweisen, wobei, vorzugsweise, Marker 17 mit Bezug auf den Eingriffszustand zu beiden Seiten eines Scheitelpunktes 18 des Begrenzungsarmes 14 vorgesehen sind. Dies ist in Figur 3 dargestellt. Bei den Markern 17 kann es sich um Metallplättchen handeln, mit denen das Begrenzungselement 8 vorzugsweise im mittleren Bereich der Schenkel 13 dotiert ist.

Der Außenkatheter 2 kann im Bereich eines proximalen Endabschnitts biegbar und vorzugsweise federelastisch sein und eine Mehrzahl von in Längsrichtung nebeneinander liegenden Ausnehmungen 19 zur Erhöhung der Biegbarkeit ausweisen. Bei der in den Figuren 1 und 2 dargestellten Ausführungsform wird der proximale Endabschnitt durch das Verlängerungsstück 15 gebildet, das die Ausnehmungen 19 und in einem vorderen Bereich die Führungsöffnungen 16 aufweist. Dies trägt zur einer hohen Stabilität des Verlängerungsstücks 15 bei.

Das Verlängerungsstück 15 besteht vorzugsweise aus einer Nickel-TitanLegierung, insbesondere aus Nitinol, das federelastische Eigenschaften aufweist und biokompatibel ist. Die Ausnehmungen 19 können dabei durch Laserschneiden in das Verlängerungsstück 15 eingebracht sein. Die Kontur einer Ausnehmung 19 ist dreieckförmig mit Bezug auf die in den Fig. 1 und 2 dargestellte Längsansicht des Verlängerungsstücks 15, wobei die eine Ausnehmung 19 begrenzenden Seitenwände 20, 21 des Verlängerungsstücks 15 in radialer Richtung nach außen aufeinander zulaufen. Durch den Abstand a der Seitenwände 20, 21 einer Ausnehmung 19 am Außenrand des Verlängerungsstücks 15 wird die maximale Abbiegbarkeit des Außenkatheters 2 an dieser Stelle festgelegt.

Die Form der Ausnehmungen 19 stellt sicher, daß das Verlängerungsstück 15 trotz der Ausnehmungen 19 beim Abbiegen ausreichende Federkräfte bzw. Rückstellkräfte ausbildet, die das Zurückfedern des Verlängerungsstücks 15 in eine Grundform sicherstellen. Es versteht sich, daß die Ausnehmungen 19 grundsätzlich auch eine andere Form aufweisen können.

Bei einer bevorzugten Ausführungsform läßt sich der proximale Endabschnitt des Außenkatheters 2 um mindestens 10°, vorzugsweise um ca. 15°, abbiegen, was die Führung der Kathetereinrichtung 1 im Körper erleichtert. Insbesondere gilt dies, wenn die Kathetereinrichtung 1 über Arterien bis zum Herzen hin geführt werden soll. In diesem Zusammenhang kann auch vorgesehen sein, daß der proximale Endabschnitt des Außenkatheters 2 bereits im Zuführzustand eine entsprechende Abbiegung aufweist, die ergänzend oder an der Stelle einer aktiv einstellbaren Abbiegung vorgesehen sein kann. Es versteht sich, daß Mittel zur Fixierung der Abbiegung des Außenkatheters 2 vorgesehen sein können, wobei, vorzugsweise, eine gerasterte Einstellbarkeit der Abbiegung in 2° bis 7°- Schritten, vorzugsweise in ca. 5°-Schritten, über entsprechende Mittel an einem Handgriff der Kathetereinrichtung 1 möglich sein kann.

Wie sich aus Fig. 3 ergibt, weist der proximale Ringabschnitt 11 des Begrenzungselementes 7 einen mittleren ringförmigen Vorsprung 22 auf, in dem die Nadel 4 bewegbar geführt ist. Das Verlängerungsstück 15 weist an seinem proximalen Ende eine Mehrzahl von Ringsegmentabschnitten 23 auf, die über Stege 24 mit einem nicht unterbrochenen Bereich 25 des Verlängerungsstücks 15 verbunden sind. Der nicht unterbrochene Bereich 25 des Verlängerungsstücks 15 ist dabei vorzugsweise nicht elastisch verformbar. Die Ringsegmentabschnitte 23 liegen stirnseitig gegen Anschlagflächen 26 des proximalen Ringabschnitts 11 und seitlich gegen Seitenflächen 27 der Schenkel 13 an. Im übrigen liegen die Ringsegmentabschnitte 23 mit den inneren Mantelflächen gegen den ringförmigen Vorsprung 22 an. Dadurch wird eine sicher Fixierung des Begrenzungselementes 7 an dem Verlängerungsstück 15 gewährleistet, ohne daß es beim Vorschieben des Innenkatheters 3 und der Nadel 4 bzw. beim Stauchen des Begrenzungselementes 7 zu einem Lösen der Verbindung zwischen dem Begrenzungselement 7 und dem Verlängerungsstück 15 kommen kann.

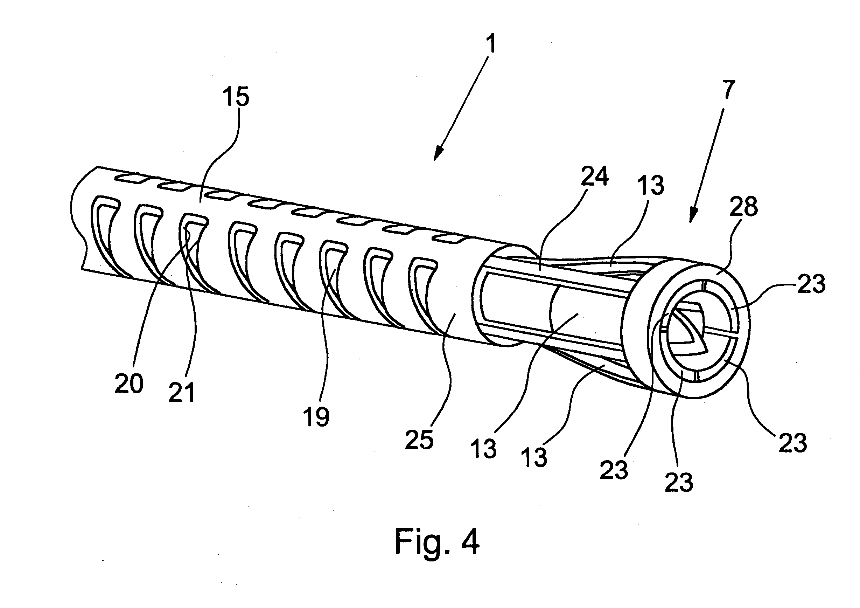

In Fig. 4 ist eine weitere Ausführungsform einer Kathetereinrichtung 1 dargestellt, die ebenfalls ein Verlängerungsstück 15 aufweist. Bei der in Fig. 4 dargestellten Ausführungsform weist das Begrenzungselement 7 jedoch einen proximalen Ringabschnitt 28 auf, in den Ringsegmentabschnitte 23 des Verlängerungsstücks 15 eingesteckt sind. Die Nadel 4 ist zwischen den Ringsegmentabschnitten 23 geführt.

Die Kanten des Verlängerungsstücks 15 können verrundet sein, vorzugsweise durch Elektropolieren, um Spitzen an der Oberfläche zu vermeiden, die zu Artefakten bei der MRT-Bildgebung führen können.

Nicht im Einzelnen dargestellt ist, daß der Außenkatheter 2 wenigstens einen nicht dargestellten Kanal für ein ebenfalls nicht dargstelltes Zugseil aufweisen kann. In diesem Zusammenhang kann ein Zugseil vorzugsweise aus Kunststoff, insbesondere Nylon, oder aus Kohlefasern, oder es kann ein ummantelter Nitinoldraht als Zugseil vorgesehen sein, um den proximalen Endabschnitt des Außenkatheters 2 aktiv, das heißt benutzergesteuert, abzubiegen. Bei einem Nitinoldraht ist zu berücksichtigen, daß es zur Erwärmung des Drahts durch die HF-Energie des MRT-Systems kommen kann. Hier ist also eine thermische Kapselung des Nitinoldrahts erforderlich, um eine Schädigung des Patienten zu verhindern. Sofern die Rückstellkräfte des Außenkatheters 2 im Bereich des proximalen Endabschnitts ausreichend hoch sind, ist lediglich ein Zugseil zum Abbiegen des vorderen Endes erforderlich, wobei die Rückstellkräfte zu einem Zurückbiegen des Außenkatheters 2 nach Entlastung des Zugseils führen. Ansonsten müssen über das Zugseil entweder Rückstellkräfte übertragbar sein, was eine entsprechende Steifigkeit des Zugseils erfordert, oder es ist ein zweites Zugseil notwendig, um den Außenkatheter 2 zurückzubiegen. Sind mehrere Zugseile vorgesehen, versteht es sich, daß auch mehrere separate Kanäle im Außenkatheter 2 vorgesehen sein können, da ein gemeinsames Lumen für mehrer Zugseile zu einer größeren Reibung und Störanfälligkeit beim Betätigen der Zugseile führt. Außerdem darf sich das Zugseil beim Abbiegen des Außenkatheters 2 nicht irreversibel auslängen bzw. plastisch verformen. Das Zugseil kann auch außerhalb des Außenkatheters 2 geführt sein, um das Abbiegen des Außenkatheters 2 in diesem Bereich zu vereinfachen. Hier muß allerdings sichergestellt sein, daß das Zugseil gegen Einschneiden in das Gewebe und/oder Gefäße des Körpers abgesichert ist.

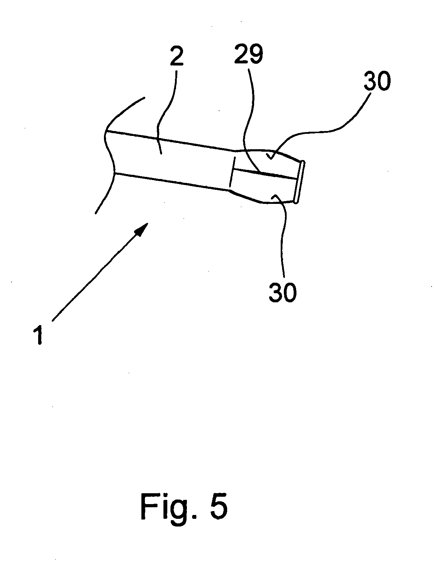

In Fig. 5 ist der proximale Endabschnitt einer weiteren Ausführungsform einer Kathetereinrichtung 1 dargestellt. Bei dieser Ausführungsform weist der Außenkatheter 2 im Zufuhrzustand an seinem proximalen Ende eine Mehrzahl von sich in Längsrichtung erstreckenden koaxial zur Katheterachse angeordneten Durchbrechungen bzw. Schlitzen 29 auf, wobei der Außenkatheter 2 im Bereich der Schlitze 29 elastisch ausgebildet ist und wobei sich der Außenkatheter 2 beim Überführen von dem Zuführzustand in den Eingriffszustand auffaltet. Im Bereich zwischen den Schlitzen 29 sind die Begrenzungsflächen 30 zur Eingriffstiefenbegrenzung vorgesehen. Beim Entlasten bzw. beim Zurückziehen des Außenkatheters von dem Gewebe sollte sich der Außenkatheter 2 vorzugsweise automatisch wieder in seine in Fig. 5 gezeigte Grundform strekken.

Die beschriebenen Kathetereinrichtungen 1 können über einen Führungsdraht, der in der Nadel 4 geführt ist, über eine Arterie an den Eingriffsort im Körper herangeführt werden. Ebensogut ist es möglich, die Kathetereinrichtung 1 über eine Schleuse aus einem flexiblen Schlauchmaterial an den Eingriffsort heranzuführen. Die Schleuse muß auf Druck belastbar sein, wobei die Schleuse vorzugsweise aus Teflon oder Silikon hergestellt sein und gegebenenfalls eine verstärkende Armierung aufweisen kann. Grundsätzlich kann die beschriebene Kathetereinrichtung 1 aber auch endoskopisch zu einem Gewebe am Eingriffsort des Körpers herangeführt werden, beispielsweise mit einem Mediastinoskop von außen an das Herzgewebe.

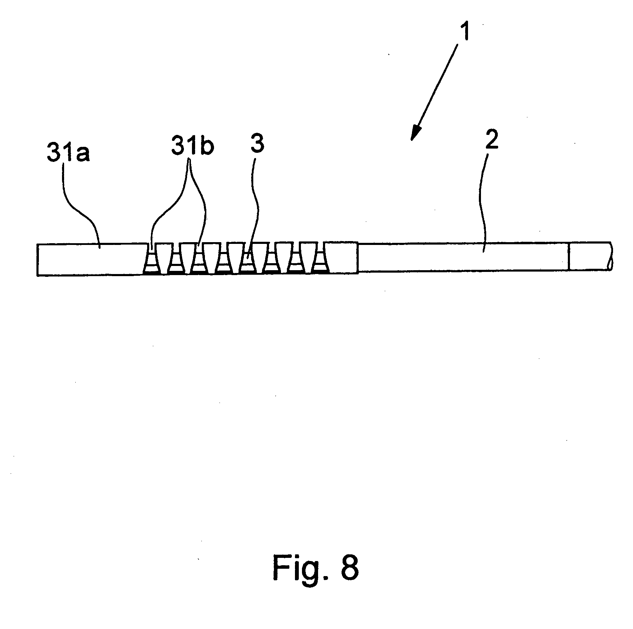

In Fig. 8 ist der vordere Teil einer vierten Ausführungsform einer erfindungsgemäßen Kathetereinrichtung 1 im Zuführzustand dargestellt. Die Kathetereinrichtung 1 weist wiederum einen Außenkatheter 2 auf, der am proximalen Ende mit einem biegbaren Verlängerungsstück 31a mit Ausnehmungen 31b verbunden ist. Das Verlängerungsstück 31 a besteht wiederum aus Nitinol. Durch die Ausnehmungen 31 b wird sichergestellt, daß das Verlängerungsstück 31a leicht biegbar ist und beim Abbiegen ausreichende Federkräfte bzw. Rückstellkräfte ausbildet, die das Zurückfedern des Verlängerungsstücks 31a bei Entlastung in die in Fig. 8 gezeigte Grundform sicherstellen. Im Inneren des Außenkatheters 2 und des Verlängerungsstücks 31a ist ein Innenkatheter 3 mit einer Nadel 4 verschiebbar geführt.

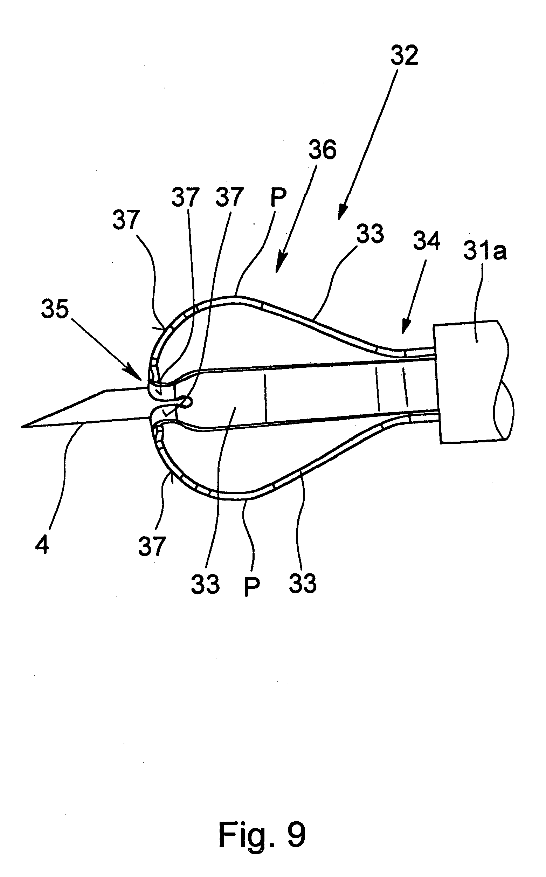

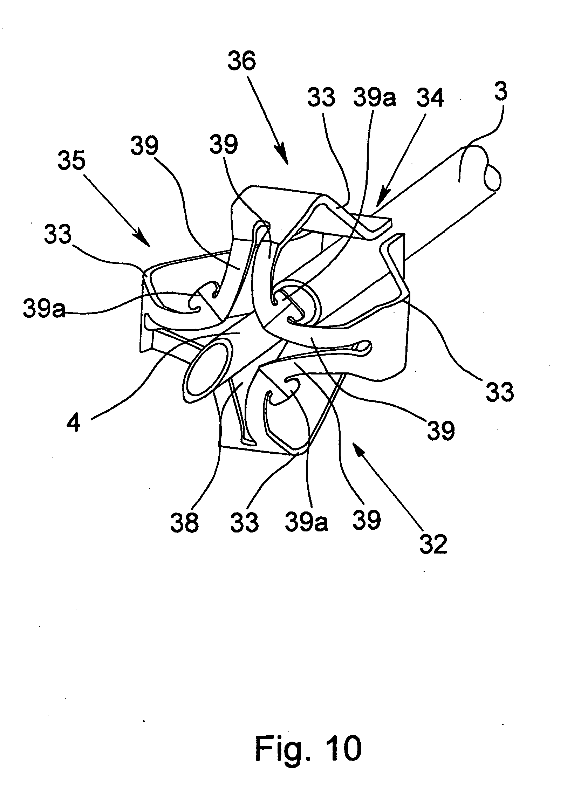

Wie sich insbesondere aus den Figuren 9 und 10 ergibt, ist ein distales Ende 34 des Begrenzungselementes 32 fest mit dem Innenkatheter 3 verbunden, so daß das Begrenzungselement 32 zusammen mit dem Innenkatheter 3 in Längsrichtung verschiebbar ist. Ein proximales Ende 35 des Begrenzungselementes 32 und die Nadel 4 sind relativ zueinander verschiebbar. Das Begrenzungselement 32 ist im Bereich zwischen dem distalen Ende 34 und dem proximalen Ende 35 elastisch ausgebildet ist, wobei das Begrenzungselement ximalen Ende 35 elastisch ausgebildet ist, wobei das Begrenzungselement 32 durch Vorschieben der Nadel 4 aus dem Außenkatheter 2 in radialer Richtung nach außen hin und/oder in proximaler Richtung expandierbar ist und Begrenzungsflächen 37 zur Begrenzung der Eingriffstiefe ausbildet. Die Begrenzungsflächen 37 können mehr oder weniger stark in Richtung zur Nadel 4 nach außen gewölbt sein, um eine Schädigung des Gewebes bei der Begrenzung der Eingriffstiefe sicher ausschließen zu können.

Die Begrenzungsflächen 37 sind auf der Außenseite von Begrenzungsschenkeln 33 vorgesehen, wobei jeder Begrenzungsschenkel 33 im Bereich zwischen den Enden 34, 35 einen elastischen Biegeabschnitt 36 aufweist. Am distalen Ende 34 sind die Begrenzungsschenkel 33 fest mit dem Innenkatheter 3 und damit mittelbar auch mit der Nadel 4 verbunden. Am proximalen Ende 35 sind die Begrenzungsschenkel 33 miteinander verbunden. Dadurch ergibt sich eine hohe Stabilität des Begrenzungselements 32 im Eingriffszustand, was eine ausreichend sichere Eingriffstiefenbegrenzung durch das Begrenzungselement 32 sicherstellt. Zwischen den am proximalen Ende 35 miteinander verbundenen Begrenzungsschenkeln 33 ist im Eingriffszustand eine Durchtrittsöffnung 38 für die Nadel 4 vorgesehen.

Die Begrenzungsschenkel 33 weisen im Eingriffszustand eine bogenförmige und im Zuführzustand eine stärker gestreckte Kontur auf. Der Begrenzungsschenkel 33 erreicht im Eingriffszustand am Punkt P die maximale Erstrekkung in radialer Richtung. Am Punkt P kann das Begrenzungselement 32 in radialer Richtung mehr als 3 mm, vorzugsweise ca. 4 mm bis 5 mm, über die Nadel 4 überstehen. In proximaler Richtung krümmt sich der Begrenzungsschenkel 33 im Eingriffszustand vom Punkt P aus in Richtung zur Nadel 4 bzw. weist einen bogenförmigen Verlauf auf, wobei die Begrenzungsfläche 37 im vorderen Teil des Begrenzungsschenkels 33 liegt und sich vom Punkt P aus bis zur Nadel 4 hin erstrecken kann. Vom Punkt P ausgehend weist der Begrenzungsschenkel 33 in distaler Richtung einen geraden geneigt zur Nadel 4 verlaufenden Schenkelabschnitt auf, der zu einer höheren Steifigkeit des Begrenzungsschenkels 33 in diesem Bereich führt, was zu einem ausreichend hohen Widerstand beim Inkontakttreten des Begrenzungselementes 32 mit dem Gewebe führt.

Im unbelasteten Eingriffszustand hat das Begrenzungselement 32 eine Ballonform, wobei durch Einziehen des Begrenzungselementes 32 in das Verlängerungsstück 31a der Expansionsgrad des Begrenzungselementes 32 verringerbar ist und wobei im Zuführzustand das Begrenzungselement 32 vollständig in dem Verlängerungsstück 31a aufgenommen sein kann. Beim Einziehen in das Verlängerungsstück 31a werden die Begrenzungsschenkel 33 zusammengedrückt, was zu einer Verlängerung des Begrenzungselementes 32 führt. Dadurch wird ein geringer Außendurchmesser des Begrenzungselementes 32 sichergestellt. Der Biegeabschnitt 36 des Begrenzungsschenkels 33 ist federelastisch, so daß die Begrenzungsschenkel 33 im Zuführzustand federbelastet bzw. vorgespannt sind. Beim Ausfahren aus dem Verlängerungsstück 31a federn die Begrenzungsschenkel 33 automatisch aus, was zu einer Verkürzung des Begrenzungselementes 32 in Längsrichtung und zur beschriebenen Ballonform führt, so wie dies in den Figuren 9, 10 dargestellt ist.

Die Ballonform des Begrenzungselementes 32 im Eingriffszustand stellt sicher, daß der behandelnde Arzt bei Inkontakttreten des expandierten Begrenzungselementes 32 mit dem Gewebe einen deutlichen Widerstand gegen ein weiteres Vorschieben der Nadel 4 spürt. Dabei werden durch die nach außen gewölbten Begrenzungsschenkel 33 im Eingriffszustand ausreichend große und in Richtung zum Gewebe hin abgerundete Anschlag- bzw. Begrenzungsflächen 37 zur Verfügung gestellt. Eine Gewebeschädigung kann damit sicher ausgeschlossen werden.

Der Begrenzungsschenkel 33 weist im Bereich des proximalen Endes 35 zwei Befestigungsstege 39 zur Befestigung mit benachbarten Begrenzungsschenkeln 33 auf. Die Befestigungsstege 39 sind nach außen gewölbt und mit Bezug auf die Mittellängsachse der Nadel 4 seitlich nach außen abgebogen, wobei die Befestigungsstege 39 von jeweils zwei benachbarten Begrenzungsschenkeln 33 miteinander verbunden sind. Im Übergangsbereich zwischen zwei Befestigungsstegen 39 von benachbarten Begrenzungsschenkeln 33 ist eine Ausformung 39a vorgesehen, die zu einer Vergrößerung der Stegbreite und damit zu einer höheren Stabilität des Begrenzungselementes 32 führt.

Das in den Figuren 9 und 10 dargestellte Begrenzungselement 32 besteht vorzugsweise aus Nitinol und ist einstückig gefertigt. Grundsätzlich könnte das Begrenzungselement 32 auch aus Kunststoff gefertigt sein.

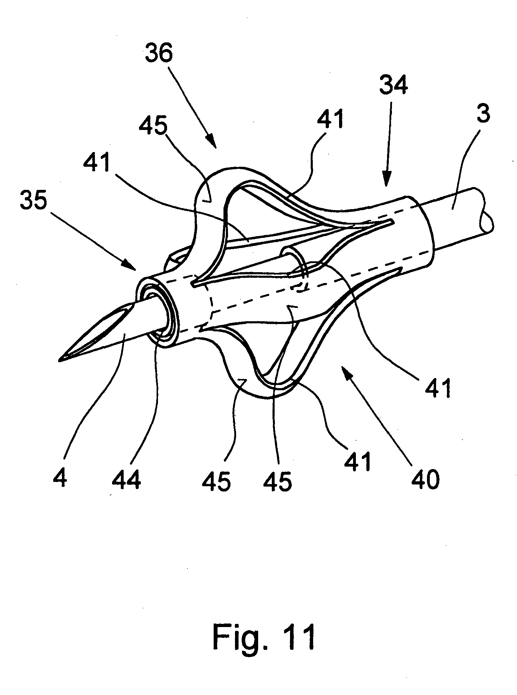

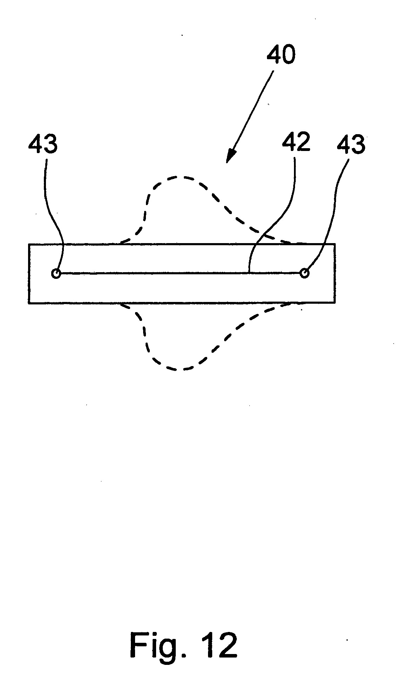

In den Figuren 11 bis 13 ist eine weitere Kathetereinrichtung 1 dargestellt. Hier ist ein im Zuführzustand rohrförmiges Begrenzungselement 40 vorgesehen, wobei das Begrenzungselement 40 eine Mehrzahl von sich in Längsrichtung erstreckenden und das Begrenzungselement 40 durchsetzenden Schlitzen 42 aufweist und wobei das Begrenzungselement 40 im Eingriffszustand im Bereich der Schlitze 42 Begrenzungsschenkel 41 zur Begrenzung der Eingriffstiefe ausbildet. Am proximalen Ende 35 und am distalen Ende 34 weist das Begrenzungselement 40 ringförmige Abschnitte auf, wobei das Begrenzungselement 40 am proximalen Ende 35 bewegbar zur Nadel 4 geführt und am distalen Ende 34 fest mit dem Innenkatheter 3 verbunden ist. Im Zuführzustand kann das Begrenzungselement 40 in den Außenkatheter 2 eingezogen sein, wobei die Begrenzungsschenkel 41 gestreckt sind und das Begrenzungselement 40 rohrförmig ist. Beim Vorschieben aus dem Außenkatheter 2 biegen sich die Begrenzungsschenkel 41 auf, wobei sich die Länge des Begrenzungselementes 40 verringert und das Begrenzungselement 40 eine Ballonform annimmt. In Fig. 12 ist gestrichelt die Form der Begrenzungsschenkel 41 im Eingriffszustand dargestellt. Deutlich läßt sich die nach außen gewölbte Form der Begrenzungsschenkel 41 im Eingriffszustand erkennen.

Vorne weist das Begrenzungselement 40 eine Durchtrittsöffnung für die Nadel 4 auf. Um das Aufbiegen der Begrenzungsschenkel 41 in die gewünschte Ballonform zu vereinfachen, sind in dem Begrenzungselement 40 Öffnungen 43 vorgesehen.

Im Zuführzustand kann der Durchmesser des Begrenzungselementes 40 ca. 1,5 mm betragen. Im expandierten Eingriffszustand kann der Außendurchmesser ca. 5 mm betragen. Im Zuführzustand kann die Nadel 4 proximal über die vordere Stirnseite des Begrenzungselementes 40 überstehen, wobei der Abstand zur Spitze der Nadel ca. 2,5 mm betragen kann. Die Breite b der Begrenzungsschenkel 41 kann ca. 1 mm betragen. Es versteht sich, daß die vorgenannten Maße nicht beschränkend für die Erfindung sind.

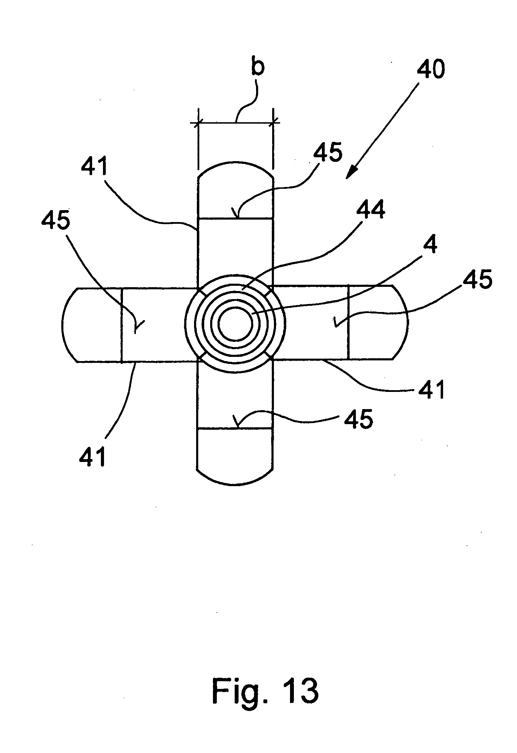

Wie sich aus den Figuren 11 und 13 ergibt, kann ein Ringabschnitt 44 in das Begrenzungselement 40 am proximalen Ende 35 eingeschoben sein, um eine leichte Bewegbarkeit des Begrenzungselementes 40 und eine sichere Führung auf der Nadel 4 zu gewährleisten.

Die Begrenzungsschenkel 41 bilden im Eingriffszustand nach außen gewölbte und abgerundete Begrenzungsflächen 45. Die Begrenzungsflächen 45 kommen im Eingriffszustand zur Begrenzung der Eingriffstiefe gegen das angrenzende Gewebe zur Anlage, wobei durch die gerundeten Oberflächen eine Verletzung des Gewebes ausgeschlossen wird.

Das Begrenzungselement 40 besteht aus Nitinol, kann grundsätzlich jedoch auch aus Kunststoff gefertigt sein.

## Patentansprüche

1. Kathetereinrichtung (1) für perkutane Eingriffe, insbesondere für Injektionen, Biopsien oder dergleichen, mit einem Außenkatheter (2) und einem Werkzeug für den Eingriff, insbesondere einer Injektionsnadel (4), einer Biopsiezange, Elektroden oder dergleichen, am proximalen Ende der Kathetereinrichtung (1), wobei das Werkzeug in einem Zuführzustand im Außenkatheter (2) aufgenommen und die Werkzeugspitze des Werkzeugs in dem Zuführzustand im Außenkatheter (2) versenkt oder mit dem proximalen Ende (6) des Außenkatheters (2) ausgefluchtet ist, wobei das Werkzeug relativ zum Außenkatheter (2) derart bewegbar ist, daß zumindest die Werkzeugspitze in einem Eingriffszustand über das proximale Ende (6) des Außenkatheters (2) übersteht und wobei ein Begrenzungselement (7) zur Eingriffstiefenbegrenzung des Werkzeugs beim Eingriff vorgesehen ist, **dadurch gekennzeichnet, daß** ein distales Ende (34) des Begrenzungselementes (32, 40) fest mit dem Werkzeug verbunden ist und/oder zusammen mit dem Werkzeug in Längsrichtung des Werkzeuges verschiebbar gelagert ist, daß ein proximales Ende (35) des Begrenzungselementes (32, 40) bewegbar zum Werkzeug ist und daß das Begrenzungselement (32, 40) im Bereich zwischen dem distalen Ende (34) und dem proximalen Ende (35) elastisch ausgebildet ist, wobei durch Vorschieben des Werkzeugs aus dem Außenkatheter (2) das Begrenzungselement (32, 40) in radialer Richtung nach außen hin und/oder in proximaler Richtung expandierbar ist und wenigstens eine Begrenzungsfläche (37, 45) zur Begrenzung der Eingriffstiefe ausbildet.

2. Kathetereinrichtung nach Anspruch 1, **dadurch gekennzeichnet, daß** das Begrenzungselement (32, 40) eine Mehrzahl von Begrenzungsschenkeln (33, 41) aufweist, wobei jeder Begrenzungsschenkel (33, 41) im Bereich zwischen den Enden (34, 35) einen elastischen Biegeabschnitt (36) mit der Begrenzungsfläche (37, 45) aufweist und die Begrenzungsschenkel (33, 41) am proximalen Ende (35) des Begrenzungselementes (32, 40) miteinander verbunden sind und wobei durch Vorschieben des Werkzeugs aus dem Außenkatheter (2) der Biegeabschnitt (36) in radialer Richtung nach außen hin und/oder in proximaler Richtung expandierbar ist und wobei, vorzugsweise, der Biegeabschnitt (36) federelastisch ist und daß die Begrenzungsschenkel (33, 41) im Zuführzustand federbelastet sind.

3. Kathetereinrichtung nach Anspruch 1 oder 2, **dadurch gekennzeichnet, daß** das proximale Ende (35) des Begrenzungselementes (32, 40) im Eingriffszustand eine Durchtrittsöffnung (38) für das Werkzeug aufweist.

4. Kathetereinrichtung nach einem der vorhergehenden Ansprüche, **dadurch gekennzeichnet, daß** zum Ausfahren des Werkzeugs aus dem Außenkatheter (2) ein innerhalb des Außenkatheters (2) relativ zum Außenkatheter (2) bewegbarer Innenkatheter (3) vorgesehen ist, wobei der Innenkatheter (3) an seinem proximalen Ende das Werkzeug aufweist und wobei das distale Ende (34) des Begrenzungselementes (32, 40) an dem Innenkatheter (3) befestigt und/oder gelagert ist.

5. Kathetereinrichtung nach einem der vorhergehenden Ansprüche, **dadurch gekennzeichnet, daß** der Außenkatheter (2) an seinem proximalen Ende mit einem biegbaren Verlängerungsstück (31a) verbunden ist, wobei das Begrenzungselement (32) im Zuführzustand vorzugsweise vollständig in das Verlängerungsstück (31a) eingezogen ist.

6. Kathetereinrichtung nach einem der vorhergehenden Ansprüche, **dadurch gekennzeichnet, daß** ein im Zuführzustand rohrförmiges Begrenzungselement (40) vorgesehen ist, wobei das Begrenzungselement (40) eine Mehrzahl von sich in Längsrichtung erstreckenden und das Begrenzungselement (40) durchsetzenden Schlitzen (42) aufweist und wobei das Begrenzungselement (40) im Eingriffszustand im Bereich der Schlitze (42) Begrenzungsschenkel (41) zur Begrenzung der Eingriffstiefe ausbildet.

7. Kathetereinrichtung nach einem der vorhergehenden Ansprüche, **dadurch gekennzeichnet, daß** das Begrenzungselement (32, 40) im Eingriffszustand am Punkt (P) seiner maximalen Ausdehnung mehr als 3 mm, vorzugsweise ca. 4 mm bis 5 mm, über das Werkzeug in radialer Richtung übersteht.

8. Kathetereinrichtung nach einem der vorhergehenden Ansprüche, **dadurch gekennzeichnet, daß** der Begrenzungsschenkel (33) am proximalen Ende (35) des Begrenzungselementes (32) zwei Befestigungsstege (39) zur Befestigung mit benachbarten Begrenzungsschenkeln (33) aufweist.

9. Kathetereinrichtung nach einem der vorhergehenden Ansprüche, **dadurch gekennzeichnet, daß** das Begrenzungselement (32, 40) einstückig ist.

10. Kathetereinrichtung (1) für perkutane Eingriffe, insbesondere für Injektionen, Biopsien oder dergleichen, mit einem Außenkatheter (2) und einem Werkzeug für den Eingriff insbesondere einer Injektionsnadel (4), einer Biopsiezange, Elektroden oder dergleichen, am proximalen Ende der Kathetereinrichtung (1), wobei das Werkzeug in einem Zuführzustand im Außenkatheter (2) aufgenommen und die Werkzeugspitze des Werkzeugs in dem Zuführzustand im Außenkatheter (2) versenkt oder mit dem proximalen Ende (6) des Außenkatheters (2) ausgefluchtet ist, wobei das Werkzeug relativ zum Außenkatheter (2) derart bewegbar ist, daß zumindest die Werkzeugspitze in einem Eingriffszustand über das proximale Ende (6) des Außenkatheters (2) übersteht und wobei ein Begrenzungselement (7) zur Eingriffstiefenbegrenzung des Werkzeugs beim Eingriff vorgesehen ist, **dadurch gekennzeichnet, daß** das Begrenzungselement (7) im Zuführzustand eine nicht-expandierte Grundform aufweist und daß das Begrenzungselement (7) durch Stauchen von der Grundform in eine expandierte Begrenzungsform im Eingriffszustand überführbar ist, wobei beim Stauchen eine relative Längenänderung des Begrenzungselementes (7) bewirkt wird und wobei das Begrenzungselement (7) beim Stauchen in radialer Richtung nach außen hin und/oder in proximaler Richtung expandiert und wenigstens eine in radialer und/oder proximaler Richtung über den Außenkatheter (2) überstehende Begrenzungsfläche (9) zur Anlage gegen ein Körpergewebe im Eingriffszustand ausbildet.

11. Kathetereinrichtung nach Anspruch 10, **dadurch gekennzeichnet, daß** das Begrenzungselement (7) beim Entlasten von der Begrenzungsform in die Grundform zurückfedert.

12. Kathetereinrichtung nach Anspruch 10 oder 11, **dadurch gekennzeichnet, daß** zum Ausfahren des Werkzeugs aus dem Außenkatheter (2) ein innerhalb des Außenkatheters (2) relativ zum Außenkatheter (2) bewegbarer Innenkatheter (3) vorgesehen ist, daß das Werkzeug am proximalen Ende (6) des Innenkatheters (3) vorgesehen ist und daß das Begrenzungselement (7) einen mit dem Innenkatheter (3) verbundenen distalen Ringabschnitt (10), einen mit dem Außenkatheter (2) verbundenen proximalen Ringabschnitt (11) und einen mittleren die Begrenzungsfläche (9) ausbildenden elastischen Bereich (12) aufweist, wobei, vorzugsweise, der elastische Bereich (12) durch eine Mehrzahl von sich in Längsrichtung erstreckenden Begrenzungsschenkeln (13) gebildet wird, wobei sich die Begrenzungsschenkel (13) beim Stauchen des Begrenzungselementes (7) in radialer Richtung nach außen biegen und die Begrenzungsflächen (9) aufweisende Begrenzungsarme (14) zur Anlage gegen ein Körpergewebe im Eingriffszustand bilden.

13. Kathetereinrichtung nach Anspruch 12, **dadurch gekennzeichnet, daß** die Begrenzungsarme (14) im Eingriffszustand im wesentlich rechtwinklig zum Außenkatheter (2) angeordnet sind.

14. Kathetereinrichtung nach einem der Ansprüche 10 bis 13, **dadurch gekennzeichnet, daß** der Außenkatheter (2) im Bereich eines proximalen Endabschnitts biegbar, vorzugsweise federelastisch, ist und, weiter vorzugsweise, eine Mehrzahl von in Längsrichtung nebeneinander liegenden Ausnehmungen (19) zur Erhöhung der Biegbarkeit aufweist, wobei, vorzugsweise die Ausnehmung (19) eine mit Bezug auf eine Seitenansicht des Außenkatheters (2) im wesentlichen dreieckförmige Kontur aufweist und seitlich durch zwei in radialer Richtung nach außen aufeinander zulaufende Seitenwände (20, 21) des Außenkatheters (2) begrenzt wird, wobei die Seitenwände (20, 21) am Außenrand des Außenkatheters (2) voneinander beabstandet sind und einen Spalt bilden.

15. Kathetereinrichtung nach Anspruch 14, **dadurch gekennzeichnet, daß** der Außenkatheter (2) mit einem den proximalen Endabschnitt bildenden biegbaren Verlängerungsstück (15) verbunden ist, wobei, vorzugsweise, das Verlängerungsstück (15) die Ausnehmungen (19) und Führungsöffnungen (16) aufweist und wobei der proximale Ringabschnitt (11) des Begrenzungselementes (7) am proximalen Ende des Verlängerungsstückes (15) gelagert ist.

## Claims

1. A catheter device (1) for percutaneous procedures, in particular for injections, biopsies or the like, having an outer catheter (2) and a tool for the procedure, in particular an injection needle (4), biopsy forceps, electrodes or the like, on the proximal end of the catheter device (1), wherein the tool in a supply state is accommodated in the outer catheter (2), and the tip of the tool in the supply state is countersunk in the outer catheter (2) or is aligned to be flush with the proximal end (6) of the outer catheter (2), wherein the tool is movable in relation to the outer catheter (2), such that, in an engaged state, at least the tip of the tool protrudes beyond the proximal end (6) of the outer catheter (2), and wherein a delimiting element (7) is provided for delimiting the depth of engagement of the tool in the procedure, **characterized in that** a distal end (34) of the delimiting element (32, 40) is fixedly connected to the tool and/or is mounted to be displaceable together with the tool in the longitudinal direction of the tool; a proximal end (35) of the delimiting element (32, 40) is movable toward the tool and the delimiting element (32, 40) is designed to be elastic in the area between the distal end (34) and the proximal end (35), wherein by advancing the tool out of the outer catheter (2), the delimiting element (32, 40) can be expanded outwardly in the radial direction and/or in the proximal direction and forms at least one delimiting surface (37, 45) for delimiting the depth of engagement.

2. The catheter device according to Claim 1, **characterized in that** the delimiting element (32, 40) has a plurality of delimiting legs (33, 41), wherein each delimiting leg (33, 41) has an elastic bending section (36) with a delimiting surface (37, 45) in the area between the ends (34, 35), and the delimiting legs (33, 41) are joined to one another at the proximal end (35) of the delimiting element (32, 40), and wherein by advancing the tool out of the outer catheter (2), the bending section (36) is expandable outwardly in the radial direction and/or in the proximal direction, and wherein the bending section (36) is preferably elastic and the delimiting legs (33, 41) are spring-loaded in the supply state.

3. The catheter device according to Claim 1 or 2, **characterized in that** the proximal end (35) of the delimiting element (32, 40) has an opening (38) for the tool in the engaged state.

4. The catheter device according to any one of the preceding claims, **characterized in that** an inner catheter (3), which is movable in relation to the outer catheter (2), is provided inside the outer catheter (2) for extracting the tool out of the outer catheter (2), wherein the inner catheter (3) has the tool on its proximal end, and wherein the distal end (34) of the delimiting element (32, 40) is attached to and/or supported on the inner catheter (3).

5. The catheter device according to any one of the preceding claims, **characterized in that** the outer catheter (2) is connected to a bendable extension piece (31a) on its proximal end, wherein the delimiting element (32) in the supply state is retracted, preferably completely, into the extension piece (31a).

6. The catheter device according to any one of the preceding claims, **characterized in that** a delimiting element (40), which is tubular in the supply state, is provided, wherein the delimiting element (40) has a plurality of slots (42) extending in the longitudinal direction and passing through the delimiting element (40), and wherein the delimiting element (40) in the engaged state forms delimiting legs (41) for delimiting the depth of engagement in the area of the slots (42).

7. The catheter device according to any one of the preceding claims, **characterized in that** the delimiting element (32, 40) in the engaged state protrudes radially by more than 3 mm, preferably approximately 4 mm to 5 mm, beyond the tool at the point (P) of its maximum extent.

8. The catheter device according to any one of the preceding claims, **characterized in that** the delimiting leg (33) has two fastening webs (39) on the proximal end (35) of the delimiting element (32) for fastening to the neighbouring delimiting legs (33).

9. The catheter device according to any one of the preceding claims, **characterized in that** the delimiting element (32, 40) is designed in one piece.

10. The catheter device (1) for percutaneous procedures, in particular for injections, biopsies or the like, having an outer catheter (2) and a tool for the procedure, in particular an injection needle (4), biopsy forceps, electrodes or the like on the proximal end of the catheter device (1), wherein the tool in the supply state is accommodated in the outer catheter (2), and the tip of the tool in the supply state is countersunk in the outer catheter (2) or is aligned to be flush with the proximal end (6) of the outer catheter (2), wherein the tool is movable in relation to the outer catheter (2), such that at least the tip of the tool in an engaged state protrudes beyond the proximal end (6) of the outer catheter (2), and wherein a delimiting element (7) is provided for delimiting the depth of engagement of the tool in engagement, **characterized in that** the delimiting element (7) in the supply state has an unexpanded basic shape, and the delimiting element (7) can be converted from the basic shape into an expanded delimiting form in the engaged state by compression, wherein a relative change in the length of the delimiting element (7) is induced in compression and wherein the delimiting element (7) is expanded outward in the radial direction and/or in the proximal direction in compression and forms at least one delimiting surface (9), which protrudes radially and/or proximally beyond the outer catheter (2) for contact with a body tissue in the engaged state.

11. The catheter device according to Claim 10, **characterized in that** the delimiting element (7) recoils from the delimiting shape into the basic shape when the pressure is released.

12. The catheter device according to Claim 10 or 11, **characterized in that** an inner catheter (3), which is movable within the outer catheter (2) in relation to the outer catheter (2), is provided for extraction of the tool out of the outer catheter (2); the tool is provided on the proximal end (6) of the inner catheter (3); and the delimiting element (7) has a distal ring section (10) connected to the inner catheter (3), a proximal ring section (11) connected to the outer catheter (2) and a central elastic area (12) forming the delimiting surface (9), wherein the elastic area (12) is preferably formed by a plurality of delimiting legs (13) extending in the longitudinal direction, wherein the delimiting legs (13) are bent outward radially in compression of the delimiting element (7) and form delimiting arms (14) having the delimiting surfaces (9) for contact with a body tissue in the engaged state.

13. The catheter device according to Claim 12, **characterized in that** the delimiting arms (14) in the engaged state are arranged essentially at a right angle to the outer catheter (2).

14. The catheter device according to any one of Claims 10 to 13, **characterized in that** the outer catheter (2) is bendable in the area of a proximal end section, preferably having spring elasticity and, more preferably, has a plurality of recesses (19) arranged side by side in the longitudinal direction to increase the bendability, wherein the recess (19) preferably has an essentially triangular contour with respect to a side view of the outer catheter (2) and is delimited laterally by two side walls (20, 21) of the outer catheter (2) tapering toward one another outwardly in the radial direction, wherein the side walls (20, 21) are spaced a distance apart from one another on the outside edge of the outer catheter (2) and form a gap.

15. The catheter device according to Claim 14, **characterized in that** the outer catheter (2) is connected to a bendable extension piece (15), forming the proximal end section, wherein the extension piece (15) preferably has the recesses (19) and guide openings (16), and wherein the proximal ring section (11) of the delimiting element (7) is supported on the proximal end of the extension piece (15).

## Revendications

1. Dispositif de cathéter (1) pour des interventions percutanées, en particulier pour des injections, des biopsies ou similaires, comprenant un cathéter externe (2) et un outil pour l'intervention, en particulier une aiguille d'injection (4), une pince à biopsie, des électrodes ou similaires, sur l'extrémité proximale du dispositif de cathéter (1), sachant que l'outil est reçu dans le cathéter externe (2) dans un état d'introduction et la pointe de l'outil est encastrée dans le cathéter externe (2) dans l'état d'introduction ou affleure avec l'extrémité proximale (6) du cathéter externe (2), sachant que l'outil est mobile par rapport au cathéter externe (2) de façon à ce qu'au moins la pointe de l'outil fasse saillie de l'extrémité proximale (6) du cathéter externe (2) dans un état d'intervention et sachant qu'un élément de délimitation (7) est prévu pour délimiter la profondeur d'intervention de l'outil lors de l'intervention, **caractérisé en ce qu'**une extrémité distale (34) de l'élément de délimitation (32, 40) est reliée fixement à l'outil et/ou est positionnée de façon à être déplacée conjointement avec l'outil, dans le sens longitudinal de l'outil, qu'une extrémité proximale (35) de l'élément de délimitation (32, 40) est mobile en direction de l'outil et que l'élément de délimitation (32, 40) est formé de façon élastique au niveau entre l'extrémité distale (34) et l'extrémité proximale (35), sachant qu'en déplaçant l'outil hors du cathéter externe (2), l'élément de délimitation (32, 40) peut se dilater en direction radiale vers l'extérieur et/ou en direction proximale et forme au moins une surface de délimitation (37, 45) pour délimiter la profondeur d'intervention.

2. Dispositif de cathéter selon la revendication 1, **caractérisé en ce que** l'élément de délimitation (32, 40) présente une pluralité de traverses de délimitation (33, 41), sachant que chacune des traverses de délimitation (33, 41) présente entre les extrémités (34, 35) une section de flexion élastique (36) avec la surface de délimitation (37, 45) et les traverses de délimitation (33, 41) sont reliées entre elles sur l'extrémité proximale (35) de l'élément de délimitation (32, 40) et sachant qu'en déplaçant l'outil hors du cathéter extérieur (2), la section de flexion (36) peut se dilater en direction radiale vers l'extérieur et/ou en direction proximale et sachant que de préférence, la section de flexion (36) est déformable élastiquement et que les traverses de délimitation (33, 41) sont contraintes par ressort dans l'état d'introduction.

3. Dispositif de cathéter selon la revendication 1 ou 2, **caractérisé en ce que** l'extrémité proximale (35) de l'élément de délimitation (32, 40) présente une ouverture de passage (38) pour l'outil dans l'état d'intervention.

4. Dispositif de cathéter selon l'une des revendications précédentes, **caractérisé en ce que** pour sortir l'outil hors du cathéter externe (2), il est prévu un cathéter interne (3) mobile à l'intérieur du cathéter externe (2) par rapport au cathéter externe (2), sachant que le cathéter interne (3) présente l'outil sur son extrémité proximale, et sachant que l'extrémité distale (34) de l'élément de délimitation (32, 40) est fixée et/ou disposée sur le cathéter interne (3).

5. Dispositif de cathéter selon l'une des revendications précédentes, **caractérisé en ce que** le cathéter externe (2) est relié à une pièce de prolongement (31a) souple sur son extrémité proximale, sachant que l'élément de délimitation (32) est rétracté entièrement dans la pièce de prolongement (31a) dans l'état d'introduction.

6. Dispositif de cathéter selon l'une des revendications précédentes, **caractérisé en ce qu'**un élément de délimitation (40) tubulaire dans l'état d'introduction est prévu, sachant que l'élément de délimitation (40) présente une pluralité de fentes (42) s'étendant dans le sens longitudinal et traversant l'élément de délimitation (40), et sachant que l'élément de délimitation (40) forme dans l'état d'intervention des traverses de délimitation (41) au niveau de la fente (42) pour délimiter la profondeur d'intervention.

7. Dispositif de cathéter selon l'une des revendications précédentes, **caractérisé en ce que** l'élément de délimitation (32, 40), dans l'état d'intervention, fait saillie de plus de 3 mm, de préférence d'environ 4 mm à 5 mm sur le point (P) de son allongement maximal, au-delà de l'outil dans le sens radial.

8. Dispositif de cathéter selon l'une des revendications précédentes, **caractérisé en ce que** la traverse de délimitation (33) présente sur l'extrémité proximale (35) de l'élément de délimitation (32), deux branches de fixation (39) pour la fixation avec des traverses de délimitation voisines (33).

9. Dispositif de cathéter selon l'une des revendications précédentes, **caractérisé en ce que** l'élément de délimitation (32, 40) est monobloc.

10. Dispositif de cathéter (1) pour des interventions percutanées, en particulier pour des injections, des biopsies ou similaires, comprenant un cathéter externe (2) et un outil pour l'intervention, en particulier une aiguille d'injection (4), une pince à biopsie, des électrodes ou similaires, sur l'extrémité proximale du dispositif de cathéter (1), sachant que l'outil est reçu dans le cathéter externe (2) dans un état d'introduction et la pointe de l'outil est encastrée dans le cathéter externe (2) dans l'état d'introduction ou affleure avec l'extrémité proximale (6) du cathéter externe (2), sachant que l'outil est mobile par rapport au cathéter externe (2) de façon à ce qu'au moins la pointe de l'outil fasse saillie de l'extrémité proximale (6) du cathéter externe (2) dans un état d'intervention et sachant qu'un élément de délimitation (7) est prévu pour délimiter la profondeur d'intervention de l'outil lors de l'intervention, **caractérisé en ce que** l'élément de délimitation (7) présente dans l'état d'introduction une forme de base non dilatée et que l'élément de délimitation (7) peut être amené de la forme de base à une forme de délimitation dilatée dans l'état d'intervention par refoulement, sachant que lors du refoulement, il se produit une modification de longueur relative de l'élément de délimitation (7) et sachant que l'élément de délimitation (7) se dilate en direction radiale vers l'extérieur et/ou en direction proximale et forme au moins une surface de délimitation (9) en saillie au-dessus du cathéter externe (2) en direction radiale et/ou proximale pour se poser contre un tissu corporel dans l'état d'intervention.

11. Dispositif de cathéter selon la revendication 10, **caractérisé en ce que** l'élément de délimitation (7) revient dans la forme de base par effet de ressort lorsqu'il est libéré de la forme de délimitation.

12. Dispositif de cathéter selon la revendication 10 ou 11, **caractérisé en ce que** pour déployer l'outil hors du cathéter externe (2), il est prévu un cathéter interne (3) mobile à l'intérieur du cathéter externe (2) par rapport au cathéter externe (2), que l'outil est prévu sur l'extrémité proximale (6) du cathéter interne (3) et que l'élément de délimitation (7) présente une section annulaire (10) distale reliée au cathéter interne (3), une section annulaire (11) proximale reliée au cathéter externe (2) et une partie élastique centrale (12) formant la surface de délimitation (9), sachant que de préférence, la partie élastique (12) est formée par une pluralité de traverses de délimitation (13) s'étendant dans le sens longitudinal, sachant que les traverses de délimitation (13) fléchissent en direction radiale vers l'extérieur lors du refoulement de l'élément de délimitation (7) et forment les bras de délimitation (14) présentant des surfaces de délimitation (9) pour se poser contre un tissu corporel dans l'état d'intervention.

13. Dispositif de cathéter selon la revendication 12, **caractérisé en ce que** les bras de délimitation (14) sont disposés essentiellement en angle droit par rapport au cathéter externe (2) en position d'intervention.

14. Dispositif de cathéter selon l'une des revendications 10 à 13, **caractérisé en ce que** le cathéter externe (2) est flexible, de préférence déformable élastiquement, au niveau d'une section d'extrémité proximale, et plus encore de préférence, présente une pluralité d'évidements (19) placés l'un à côté de l'autre dans le sens longitudinal pour augmenter la flexibilité, sachant que de préférence, l'évidement (19) présente un contour essentiellement triangulaire par rapport à une vue latérale du cathéter externe (2) et est délimité latéralement par deux parois latérales (20, 21) du cathéter externe (2) partant l'une sur l'autre vers l'extérieur dans le sens radial, sachant que les parois latérales (20, 21) sont à distance l'une de l'autre sur le bord extérieur du cathéter externe (2) et forment une fente.

15. Dispositif de cathéter selon la revendication 14, **caractérisé en ce que** le cathéter externe (2) est relié à une pièce de prolongement (15) flexible formant la section d'extrémité proximale, sachant que de préférence, la pièce de prolongement (15) présente les évidements (19) et des ouvertures de guidage (16) et sachant que la section annulaire proximale (11) de l'élément de délimitation (7) est disposée sur l'extrémité proximale de la pièce de prolongement (15).
